# EUROPÄISCHE PATENTANMELDUNG

(11) **EP 2 913 366 A1**
(43) Veröffentlichungstag der Anmeldung: **02.09.2015**
(21) Anmeldenummer: 14157248.7
(22) Anmeldetag: 28.02.2014
(51) Int. Cl.: C09B 61/00, C08B 37/16

(54) **Anthocyanidin-Komplex**

(71) Anmelder: SapioTec GmbH, 97082 Würzburg (DE)
(72) Erfinder: Roewer, Norbert, Prof. Dr., 97082 Würzburg (DE); Broscheit, Jens, Dr. med., 97209 Würzburg (DE)
(74) Vertreter: Glawe, Delfs, Moll

(57) **Zusammenfassung**

Gegenstand der Erfindung ist ein Komplex aus einem Anthocyanidin und einem methylierten ß-Cyclodextrine, der als wässrige Lösung sowie als Feststoff formuliert werden kann, sowie ein Verfahren zur Herstellung eines solchen Komplexes. Erfindungsgemäße Komplexe sind lagerstabil und lassen sich gut in wässrige Lösung formulieren.

## Beschreibung

Die Erfindung betrifft einen Komplex aus einem Anthocyanidin und einem Cyclodextrin.

Anthocyanidine sind zymochrome Farbstoffe, die in den meisten höheren Landpflanzen vorkommen. Anthocyanidine sind zuckerfrei (Aglycone) und eng verwandt mit dem zuckerhaltigen Anthocyanen. Anthocyanidine sind Farbstoffe und besitzen antioxidative Eigenschaften.

Aus WO 2013/144297 A1 ist es bereits bekannt, Anthocyanidine mit Sulfoalkylether-β-Cyclodextrinen zu komplexieren.

Der Erfindung liegt die Aufgabe zu Grunde, Anthocyandine in gut handhabbarer, formulierbarer und lagerstabiler und verhältnismäßig konzentrierter Form zur Verfügung zu stellen.

Gelöst wird diese Aufgabe durch einen Komplex aus einem Anthocyanidin und einem methylierten ß-Cyclodextrin.

Zunächst sein einige im Rahmen der Erfindung verwendete Begriffe erläutert.

Anthocyanidine weisen die nachfolgend wiedergegebene Grundstruktur auf.

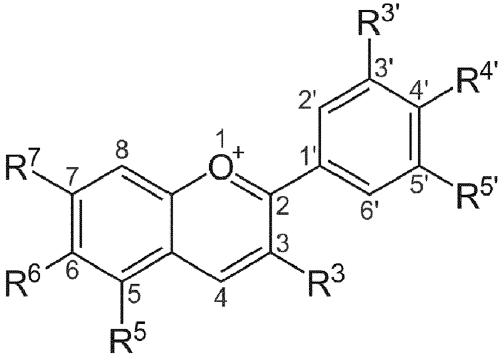

Die Substituenten in dieser Formel sind ausgewählt aus der Gruppe bestehend aus Wasserstoff, Hydroxygruppe und Methoxygruppe.

Cyclodextrine sind zyklische Oligosaccharide aus α-1,4-glykosidisch verknüpften Glukosemolekülen. β-Cyclodextrin besitzt sieben Glukoseeinheiten. Bei einem methylierten β-Cyclodextrin sind Hydroxygruppen der Glukoseeinheit mit Methylgruppen versehen. Erfindungsgemäß ist in der Regel lediglich ein Teil der 21 Hydroxygruppen eines β-Cyclodextrins methyliert.

Die Herstellung von methylierten β-Cyclodextrinen ist dem Fachmann geläufig, entsprechende Produkte sind bspw. von der Wacker Chemie unter der Bezeichnung Cavasol® erhältlich.

Die Erfindung hat erkannt, dass sich überraschenderweise mit methylierten β-Cyclodextrinen Anthocyanidine wie Delphinidin in weit höherer Konzentration komplexieren lassen als im Stand der Technik mit anderen Cyclodextrinen. Dies ist insbesondere deshalb überraschend, weil beispielsweise in der WO 2013/144297 A1 Komplexierungsversuche mit einer Reihe von Cyclodextrinen durchgeführt wurden, bei denen die Konzentration bzw. Beladung des Komplexes mit Anthocyanidin um Größenordnungen geringer war.

Die vorliegende Erfindung ermöglicht es somit, Anthocyanidine in hoher Konzentration und damit hohen Dosierungen bevorzugt in wässriger oder wasserlöslicher Form zur Verfügung zu stellen und damit auf einfache Weise einer in vivo Administration, beispielsweise eine Administration i.v. zugänglich zu machen. Von besonderem Vorteil der Erfindung ist ferner, dass methylierte Cyclodextrine, insbesonder RAMEB, in den zum Einsatz kommenden Konzentrationen nicht oder allenfalls geringfügig toxisch sind, insbesondere in vivo keine oder allenfalls eine geringfügige und unbedenkliche Hämolyse auslösen.

Bevorzugt beträgt der der Substitutionsgrad des β-Cyclodextrins mit Methylgruppen 10 bis 15, vorzugsweise 11 bis 14, weiter vorzugsweise 12 bis 13.

Besonders bevorzugt wird als methyliertes β-Cyclodextrin RAMEB (randomly methylated β-Cyclodextrin) eingesetzt. Es handelt sich um ein statistisch methyliertes β-Cyclodextrin mit einem Substitutionsgrad von etwa 1,8 Methylgruppen pro Zuckereinheit bzw. 12,5 Methylgruppen pro Cyclodextrinring (DS degree of substitution ungefähr 12,5). RAMEB ist kommerziell erhältlich bspw. von Wacker Chemie unter der Bezeichnung Cavasol® W7 M Pharma.

Die erfindungsgemäß komplexierten Anthocyanidine sind bevorzugt ausgewählt aus der Gruppe bestehend aus Aurantinidin, Cyanidin, Delphinidin, Europinidin, Luteolinidin, Pelargonidin, Malvidin, Peonidin, Petunidin und Rosinidin.

Die chemische Struktur entspricht der oben wiedergegebenen Formel I mit dem folgenden Substitutionsmuster

| | R^{3'} | R^{4'} | R^{5'} | R³ | R⁵ | **R**⁶ | R⁷ |
|---|---|---|---|---|---|---|---|
| Aurantinidin | -H | -OH | -H | -OH | -OH | -OH | -OH |
| Cyanidin | -OH | -OH | -H | -OH | -OH | -H | -OH |
| Delphinidin | -OH | -OH | -OH | -OH | -OH | -H | -OH |
| Europinidin | -OCH₃ | -OH | -OH | -OH | -OCH₃ | -H | -OH |
| Luteolinidin | -OH | -OH | -H | -OH | -OH | -H | -OH |
| Pelargonidin | -H | -OH | -H | -OH | -OH | -H | -OH |
| Malvidin | -OCH₃ | -OH | -OCH₃ | -OH | -OH | -H | -OH |
| Peonidin | -OCH₃ | -OH | -H | -OH | -OH | -H | -OH |
| Petunidin | -OH | -OH | -OCH₃ | -OH | -OH | -H | -OH |
| Rosinidin | -OCH₃ | -OH | -H | -OH | -OH | -H | -OCH₃ |

Besonders bevorzugt ist im Rahmen der Erfindung ein Komplex mit Delphinidin.

Gegenstand der Erfindung ist ferner eine wässrige Lösung eines erfindungsgemäßen Komplexes.

Ein weiterer Gegenstand der Erfindung ist ein Verfahren zur Herstellung eines erfindungsgenäßen Komplexes sowie einer entsprechenden wässrigen Lösung, mit den Schritten:
a)Herstellen einer wässrigen Lösung des methylierten ß-Cyclodextrins,
b)Zugabe des Anthocyanidins und Vermischen zur Herstellung des Komplexes.

In Schritt a) wird bevorzugt eine wässrige Lösung hergestellt, die 10 bis 60 Gew.-%, weiter vorzugsweise 20 bis 50 Gew.-%, weiter vorzugsweise 30 bis 50 Gew.-% des verwendeten Cyclodextrins enthält.

Besonders bevorzugt ist es im Rahmen der Erfindung, wenn der pH-Wert der wässrigen Lösung während oder nach, bevorzugt jedoch vor der Zugabe des Anthocyanidins, bevorzugt Delphinidins, auf einen pH-Wert von 7 oder weniger, vorzugsweise 6 bis 7 eingestellt wird. Es hat sich gezeigt, dass bei diesem pH-Wert sich eine höhere Konzentration des Komplexes in wässriger Lösung einstellen lässt.

Bevorzugt beträgt die Konzentration des Anthcyanidins, berechnet als Chlorid, wenigstens 10 mg/ml, weiter vorzugsweise wenigstens 20 mg/ml, weiter vorzugsweise wenigstens 50 mg/ml, weiter vorzugsweise wenigstens 80 mg/ml. Konzentrationen von etwa 100 mg/ml können problemlos erreicht werden.

Ebenfalls Gegenstand der Erfindung ist ein Feststoff enthaltend einen Komplex aus einem Anthocyanidin und einem methylierten β-Cyclodextrin, erhältlich durch Entfernen des Lösungsmittels aus einer wässrigen Lösung. Das Entfernen des Lösungsmittels aus der wässrigen Lösung kann durch dem Fachmann geläufige Verfahren wie beispielsweise Gefriertrocknung (Lyophilisation) erfolgen. Dieser erfindungsgemäße Feststoff ist langfristig lagerstabil und kann problemlos mit Wasser wieder zu einer wässrigen und damit in vivo administrierbaren Lösung angesetzt werden. Sowohl die erfindungsgemäße wässrige Lösung als auch der Feststoff besitzen eine hohe Lagerstabilität.

Im Rahmen der erfindungsgemäßen Herstellung kann das Vermischen der Bestandteile der wässrigen Lösung durch Rühren geschehen, bevorzugte Zeiträume für das Vermischen sind 2 bis 20 h. Bevorzugt wird im Dunkeln gearbeitet, um eine lichtinduzierte Oxidation zu vermeiden.

Ausführungsbeispiele der Erfindung werden nachfolgend erläutert.

### 1. Eingesetzte Materialien:

RAMEB wurde von Wacker Chemie und Delphinidinchlorid von der Firma Extrasynthese erworben.

### 2. Bestimmung des Delphinidingehalts

Zur Bestimmung des Gehalts von Delphinidinchlorid in den delphinidinhaltigen Zusammensetzungen wurde ein Reverse phase HPLC-Verfahren verwendet. Dabei wurden folgende Reagenzien eingesetzt:

### Gereinigtes Wasser

Methanol für die Chromatographie Armeisensäure, p. a.
1 M Salzsäure als volumetrische Lösung.

Als Säule wurde eine Waters X Bridge™ C18,35 µl, 150 mm x 4,6 mm verwendet.

Die mobilen Phasen waren wie folgt:
Kanal A: Wasser 950 ml, Methanol 50 ml, Armeisensäure 10 ml
Kanal B: Wasser 50 ml, Methanol 950 ml, Armeisensäure 10 ml

Folgendes Gradientenprogramm wurde verwendet:

| Zeit [min] | Prozent Kanal B |
|---|---|
| 0 | 0 |
| 5 | 0 |
| 25 | 60 |
| 30 | 100 |

Stoppzeit: 35 min
Nachlaufzeit (posttime): 8 min
Flussrate: 1 ml/min
Injektionsvolumen: 20 µl
Säulentemperatur: 30°C +/- 2°C
UV-Vis Detektor: 530 µm für den Assay, 275 µm zur Detektion von Verunreinigungen
Integrator: Fläche

Lösungen und Probenvorbereitung:
- Verdünnungslösung 1:: Mischung aus 100 ml Methanol und 2,6 ml 1 M HCL
- Verdünnungslösung 2:: Mischung aus 100 ml 40 prozentiger Methanol und 2,6 ml 1 M HCL

Kalibrierungslösung: Eine Referenzlösung von Delphinidin wurde durch Einwiegen von 10 mg Delphinidinchlorid in einen 10 ml Kolben und Lösen in Verdünnungslösung 1 hergestellt. Nach dem Lösen wurde ungefähr 10fach verdünnt mit Verdünnungslösung 2 zur Herstellung einer ungefähren Konzentration von 0,1 mg/ml.

Die Kontrollkalibrierungslösung wurde auf die gleiche Art und Weise hergestellt. Die Kalibrierungslösungen wurden sofort mittels HPLC analysiert, da Delphinidinchlorid in Lösung instabil ist.

### Herstellung der Prüflösungen:

Zur Bestimmung des Delphinidingehalts erfindungsgemäß hergestellter Feststoffe (Herstellung siehe weiter unten) wurde etwa 50 mg dieser Zusammensetzung in einem 10 ml Kolben eingewogen. Anschließend wurde in Verdünnungslösung 2 gelöst und mit der gleichen Verdünnungslösung 2 weiter verdünnt bis zur Einstellung einer ungefähren Delphinidinkonzentration von 0,1 mg/ml.

Die Bestimmung des Delphinidingehalts in den Proben wurde berechnet unter Zuhilfenahme der Agilent ChemStation Software unter Verwendung der Kalibrierung mit dem beschriebenen externen Standard.

### Beispiel 1

Komplexierung von Delphinidin mit RAMEB.

Es wurde Lösungen von 40 Gew.-% RAMEB in Wasser hergestellt.

1 ml der wässrigen Cyclodextrinlösung wurde in einen Glaskolben gefüllt. Anschließend wurde 250 mg Delphinidinchlorid zugegeben.

Die Suspension wurde für 4 h bei 30° C im Dunkeln gerührt. Anschließend wurde filtriert durch einen Membranfilter mit 0,8 µm Porengröße.

### Beispiel 2

### Herstellung eines erfindungsgemäßen Feststoffs

Die Lösung gemäß Beispiel 1 wurde gefroren und anschließend gefriergetrocknet bei -48°C und einem Druck von etwa 10,3 Pa (77 mTorr). Man erhielt 0,36 g eines Feststoffs mit einem Delphinidingehalt von 31,1 Gew.-%.

Dieser Feststoff stellt Delphinidin in hoher Konzentration in lagerfähiger und ohne weiteres in vivo administrierbaren Form zur Verfügung. Der Gehalt des Komplexes an Delphinidin ist weit höher als im Stand der Technik.

## Patentansprüche

1. Komplex aus einem Anthocyanidin und einem methylierten β-Cyclodextrin.

2. Komplex nach Anspruch 1 oder 2, **dadurch gekennzeichnet, dass** der Substitutionsgrad des β-Cyclodextrins mit Methylgruppen 10 bis 15, vorzugsweise 11 bis 14, weiter vorzugsweise 12 bis 13 beträgt.

3. Komplex nach Anspruch 2, **dadurch gekennzeichnet, dass** das methylierte β-Cyclodextrin RAMEB ist.

4. Komplex nach einem der Ansprüche 1 bis 3, **dadurch gekennzeichnet, dass** die Anthocyanidine ausgewählt sind aus der Gruppe bestehend aus Aurantinidin, Cyanidin, Delphinidin, Europinidin, Luteolinidin, Pelargonidin, Malvidin, Peonidin, Petunidin und Rosinidin.

5. Komplex nach Anspruch 4, **dadurch gekennzeichnet, dass** das Anthocyanidin Delphinidin ist.

6. Wässrige Lösung eines Komplexes nach einem der Ansprüche 1 bis 5.

7. Wässrige Lösung nach Anspruch 6, **dadurch gekennzeichnet, dass** die Konzentration des Anthcyanidins, berechnet als Chlorid, wenigstens 10 mg/ml, weiter vorzugsweise wenigstens 20 mg/ml, weiter vorzugsweise wenigstens 50 mg/ml, weiter vorzugsweise wenigstens 80 mg/ml beträgt.

8. Feststoff enthaltend einen Komplex aus einem Anthocyanidin und einem methylierten β-Cyclodextrin, erhältlich durch Entfernen des Lösungsmittels aus einer wässrigen Lösung nach gemäß einem der Ansprüche 6 oder 7.

9. Verfahren zur Herstellung eines Komplexes aus einem Anthocyanidin und einem methylierten β-Cyclodextrin, mit den Schritten:
a. Herstellen einer wässrigen Lösung des methylierten β-Cyclodextrins,
b. Zugabe des Anthocyanidins und Vermischen zur Herstellung des Komplexes.

10. Verfahren nach Anspruch 9, **dadurch gekennzeichnet, dass** die in Schritt a) hergestellte Lösung 10 bis 60 Gew.-%, vorzugsweise 20 bis 50 Gew.-%, weiter vorzugsweise 30 bis 50 Gew.-% des methylierten β-Cyclodextrins enthält.

11. Verfahren nach Anspruch 9 oder 10, **dadurch gekennzeichnet, dass** das Vermischen in Schritt b) über einen Zeitraum von 2 bis 20 h stattfindet.
